# EUROPEAN PATENT SPECIFICATION

(11) **EP 3 615 009 B1**
(45) Date of publication and mention of the grant of the patent: **07.06.2023**
(21) Application number: 18721748.4
(22) Date of filing: 27.04.2018
(51) Int. Cl.: A61K 9/48, A61K 31/57, A61P 5/34, A61P 5/30

(54) **HORMONE SOFTGEL CAPSULES AND A PROCESS FOR THE PREPARATION THEREOF**
HORMON-SOFTGELKAPSELN UND VERFAHREN ZUR HERSTELLUNG DAVON
CAPSULES MOLLES D'HORMONE ET PROCÉDÉ DE PRÉPARATION DE CELLES-CI

(30) Priority: 28.04.2017 EP 17382230
(43) Date of publication of application: 04.03.2020
(73) Proprietor: Chemo Research, S.L., 28050 Madrid (ES)
(72) Inventor: RONCHI, Celestino, 20124 Milano (IT); MOYA ORTEGA, María Dolores, 24008 León (ES); RIBEIRO, Andreza Maria, 24193 León (ES)
(74) Representative: Ponti & Partners, S.L.P
(86) International application number: PCT/EP2018/060947
(87) International publication number: WO 2018/197698

(56) References cited:
- EP-A2- 0 393 539
- US-A- 6 165 491
- US-A1- 2005 004 076
- US-A1- 2010 330 168
- Anonymous: "UTROGESTAN 100 mg and 200 mg soft capsules (Progesterone)", , June 2002 (2002-06), page 1, XP055407543, Retrieved from the Internet: URL:http://www.besins-healthcare.com/pdf/U trogestan 200mg PIL (English).pdf [retrieved on 2017-09-18]

## Description

### Field of the invention

The present invention belongs to the pharmaceutical hormone softgel capsules field, as well as to a process for the preparation thereof. In particular, the present invention relates to novel softgel capsules of micronized progesterone, as well as to their use in the pharmaceutically industry for producing substances and end dosage forms.

### Background of the invention

The drug to be delivered is progesterone.

Progesterone, enormously used in humans as well as veterinary medicines, is the most widely occurring progestogen type of hormone. Though progesterone has very low solubility in water. The main use of progesterone is in menopausal hormone replacement therapies and oral contraceptives. Progesterone is secreted by ovaries, placenta and adrenal glands and is essential during luteal phase to sustain pregnancy.

Progesterone could be administered orally, vaginally, or by intramuscular injection. Progesterone administered orally demonstrated lower bioavailability due to the first-liver-pass effect, which calls for the use of higher doses that give rise to a fairly large number of side effects such as somnolence and sedation, which are also associated with a lower pregnancy rate. There is increasing evidence that vaginal and intramuscular progesterone are at least equally effective, considering the rate of biochemical and clinical pregnancies as well as their outcomes. However, through the use of vaginal progesterone, reiterated painful application of IM injections and their complications, such as local soreness, abscesses, and inflammatory reactions were avoided.

The use of vegetable oils to formulate soft capsules has been reported in some commercial products. The vegetable oils can be selected from a long list including sunflower seed, Brazil nut, olive, sesame, safflower, sunflower, and peanut oil. Peanut oil, used of in the oral administration, can generate different forms of allergic reactions.

The European patent EP1443940B1 discloses formulations wherein the peanut oil has been replaced by other oils such as sunflower oil, olive oil, sesame oil, rapeseed oil or almond oil, which make it possible to suppress the risks of allergic reactions of peanut oil while at the same time conserving all the physico-chemical characteristics of the progesterone formulation. These vegetable oils have a high content in polyunsaturated fatty acids. However, it is stated that these formulations still contain a certain proportion of soybean lecithin (included as essential element to avoid sedimentation of progesterone within the formulation), which is also known to possess considerable allergenic properties. This lipid is said to be capable of inducing an immune response that carries with it the risk of hypersensitive reactions (anaphylactic shock, urticaria).

In the same direction, EP2269592 discloses capsules of progesterone and safflower oil type II devoid of soybean lecithin and compares the behaviour of progesterone in different oils, including peanut oil. This European Patent is focused to overcome problems derived from existing commercial formulations comprising peanut oil, which is known to possess allergenic properties and to cause considerable hypersensitivity reactions. The peanut oil + soybean lecithin present in RLD is replaced by safflower oil type II. The reason given relies on the nature of safflower oil. Safflower oil, despite the fact of having a similar viscosity value to peanut oil, shows a high content in monounsaturated fatty acids (mainly oleic acid: 65-85%) and low content in polyunsaturated fatty acids (mainly linoleic acid: 5-25%), in comparison with peanut oil which has a higher content in polyunsaturated fatty acids (oleic acid: 38-63% and linoleic acid: 18-42%)*[*Data from Grasas y Aceites Vol. 51. Fasc.1-2 (2000), 74-96].

Viscosity of safflower oil is slightly higher than that of peanut oil and peanut oil + soybean lecithin. However, values of solubility and sedimentation of progesterone in safflower oil are better than those shown for compositions of peanut oil +lecithin.

In view of the state of the art, there is still the need to provide an improved softgel capsule of progesterone having a good dispersion, antioxidant value, stability, nutritional value, and low risks of allergenic reactions.

### Summary of the invention

The present invention was made in view of the prior art described above.

The aim of the present invention is to elaborate pharmaceutical compositions for preparing softgel capsules from progesterone on the basis of micronized progesterone in combination with grapeseed oil, and a process for the preparation thereof. Advantageously, the hormone softgel capsule according to the present invention does not employ peanut oil.

The technical result achieved during the realization of the present invention is a reduced allergenic reaction of oral and vaginal dosage preparations on the basis of use a micronized hormone in combination with grapeseed oil.

Particularly, the grape seed oil (GSO) as an extract of the grape seed has many uses ranging from cooking (as a food additive), cosmetics and in controlling several diseases and wound healing potential. Nowadays, many scientific researchers have revealed that the GSO has several health benefits and is considered as a good and potent antioxidant compound for its contents of polyphenols, flavonoids, unsaturated fatty acids, and vitamin E. Grape seed contains around 13% of oil with high level of linoleic and oleic acids. It has been reported a total polyphenol content ranging from 59 to 115.5 mg/g as gallic acid in grape seeds. Grape seed oil contains 399.785 mg/kg vitamin (E) depending on variety and environmental conditions. The biological activity of tocopherols and tocotrienols are mainly attributed to their antioxidant activity in inhibiting lipid peroxidation in biological membranes. Alpha-tocopherol is the most active form of vitamin E and accounts for almost all vitamin E activity in living tissue. Medicinally tocopherol plays an important role in the prevention of cancer, inflammatory activities and cardiovascular disease. The beneficial effects of grape seed is due not only to their high degree of unsaturation, but also to their antioxidants components such as tocopherols which may serve as dietary sources of natural antioxidants preventing diseases and promoting human health.

Oil content of grape seed strongly depends on grape variety, though the usual range is 10-16% of dry weight. It consists mainly of triglycerides and the fatty acids composition is adjusted to the following value: 0-0.2% myristic acid (C14:0), 7-13% palmitic acid (C16:0), 3-6% stearic acid (C18:0), 0-0.9%, palmitoleic acid (C16:1), 14-25%; oleic acid (C18:1), 61-73% linoleic acid (C18:2), 0-0.6% linolenic acid (C18:3). The high content in unsaturated fatty acids (around 85-90%) makes it high quality nutritional oil.

Surprisingly, in pharmaceutical softgel capsules according the present invention, the grape seed oil, which is a vegetable oil with high content of polyunsaturated fatty acids, does not require soybean lecithin contrary to the teachings of EP1443940, wherein vegetable oils with high content of polyunsaturated fatty acids require to be used with soybean lecithin as essential component in the formulation.

Furthermore, the pharmaceutical hormone softgel capsule according to the invention has an additional advantageous effect due to the nature of the selected grape seed oil, which has a beneficial effect for health in general, and particularly decreases the blood cholesterol. Micronization is a conventional technique for the particle size reduction and is a commonly used method for increasing solubility of BCS class II drugs. It has been well explained that solubility, dissolution and gastrointestinal permeability are fundamental parameters that control rate and extent of drug absorption and its bioavailability. The term micronization usually refers to the reduction of average particle diameters to the micrometer range, but can also describe further reduction to the nanometer scale. Common applications include the production of active chemical ingredients, foodstuff ingredients, and pharmaceuticals. Micronization does not increase the equilibrium solubility of the drug itself but it increases the dissolution rate by increasing the surface area to drug ratio by which the active ingredient can dissolve or diffuse from the drug particles. These active ingredients need to be micronized to increase efficacy. Particles with reduced diameters have higher dissolution rates, which increases efficacy. The problem posed by the present invention above has been solved by a group of inventions by creating pharmaceutical dosage preparations for the preparation of softgel capsules of progesterone in combination with grapeseed oil, which are capable of reducing allergic reactions, while having a good dissolution rate in the administration thereof.

The present invention, in a first aspect, provides pharmaceutical compositions for the preparation of softgel capsule dosage forms comprising progesterone on the basis of an outer soft capsule shell and an inner hormone formulation, which is characterized in that:
- the outer soft capsule shell comprises gelatin, and
- the inner hormone formulation comprises micronized progesterone in combination with grapeseed oil.

The present invention also relates to a process for the preparation of such pharmaceutical softgel capsules.

Advantageously, the hormone softgel capsule according to the invention does not use peanut oil, thus reducing the risks of allergenic reactions derived from its use in mammals and, in addition, shows dissolution rates higher than of that of the prior art (Utrogestan^{®}).

The authors of the present invention have designed a novel softgel capsule of a women health care hormone that provides improved dispersion of said hormone, a high antioxidant value, an adequate stability of the final formulation, together with a high nutritional value. Moreover, the novel softgel capsule according to the first aspect, which can be free of soybean lecithin, provides additional advantages that will be explained herein.

Advantageously, the hormone softgel capsule according to the present invention does not use peanut oil, thus reducing the risks of allergenic reactions derived from its use in mammals.

In a variant, the women health care hormone formulation further comprises soybean lecithin. In this variant, the softgel capsule is characterized in that:
- the outer soft capsule shell comprises gelatin, and
- the inner hormone formulation comprises micronized progesterone in combination with grapeseed oil and soybean lecithin.

This pharmaceutical dosage form is particularly advantageous for using in mammals having hypersensitive to the allergens.

For the purpose of the present invention, the women health care hormone is micronized progesterone.

In a preferable embodiment, the inner hormone formulation comprises micronized progesterone 20-60% by weight, grapeseed oil 39-80% by weight, and soybean lecithin 1% by weight.

In a still more preferable embodiment, the inner hormone formulation comprises micronized progesterone 40% by weight, grapeseed oil 59.6% in weight, and soybean lecithin 0.4% by weight.

It is of general knowledge of a skill person in the art the fact that additional pharmaceutically acceptable excipients and/or vehicles can be added into the hormone formulation. In this case, the percentages of micronized progesterone, grapeseed oil, and soybean lecithin when it is present, would be slightly decreased from the ones stated above.

In second variant, the women health care hormone formulation is free of soybean lecithin.

In this variant, the softgel capsule is characterized in that:
- the outer softcapsule shell comprises gelatin, and
- the inner hormone formulation comprises micronized progesterone
in combination with grapeseed oil, being the hormone formulation free of soybean lecithin. This pharmaceutical dosage form is particularly advantageous for using in mammals having higher hypersensitive to the allergens.

In an embodiment, the inner hormone formulation comprises micronized progesterone 20-60% by weight in combination with grapeseed oil 40-80% by weight.

A typical softgel capsule shell composition includes gelatin, a plasticizer such as glycerin, an opacifying/coloring agent in order to give the desired color to the capsule and water. The water is principally evaporated during the drying stage of the manufacturing process.

Although, the manufacturing of softgel capsule shell is well known in the state of the art, the authors of the present invention have further designed an improved softgel capsule shell.

Thus, the present invention also provides an outer soft capsule shell composition comprising gelatin between 36 to 56% by weight, glycerine between 13.5 to 23.5% by weight, titanium oxide between 0.3 to 0.7% by weight, and water between 25 to 45% by weight, being the sum of said shell's components 100%.

In a preferable embodiment, the outer soft capsule shell composition comprises gelatin between 40 to 45% by weight, glycerine between 15 to 20% by weight, titanium oxide between 0.4 to 0.6% by weight, and water between 32 to 42% by weight, being the sum of said shell's components 100%.

A second object of the present invention is to provide a practical process capable of preparing the women health care hormone softgel capsule according to the first object of the invention. The encapsulating process according to the second aspect of the invention can be scalable at industrial scale, and is capable of preparing hormone softgel capsules in a lower time and lower costs of the starting materials.

The process for preparing the softgel capsule of micronized progesterone according to the first aspect of the present invention comprises selecting micronized progesterone and separately, preparing a gelatin mass for making an outer softgel capsule shell and a filler suspension as hormone formulation, characterized in that the process comprises the following steps:
- encapsulating the previously prepared filler suspension into the previously prepared softgel capsule shell,
- drying the encapsulated filler suspension at controlled conditions of temperature and humidity, and
- confirming moisture content and hardness of the thus prepared softgel capsules.

The softgel capsule is composed of a capsule-shaped shell and a filler suspension.

In order to prepare the capsule-shaped shell of gelatin, the above capsule shell composition comprising gelatin, glycerine, titanium oxide, and water is processed according to the following steps:
a) preparing a gelatin mass by:
   - heating gelatin in water to form a gel mass,
   - deaerating the gelatin mass,
   - adding into the gelatin mass an opacifying or colouring agent and glycerine to form a solution,
   - deaerating the solution, and
   - confirming viscosity of the solution, and adjusted if necessary to a value from 7 Pa.s (7000cP) and 8.5 Pa.s (8500cP),
b) preparing molded halves of capsule-shaped shell from the solution obtained in step
   a) by using a suitable equipment.

A lubricant can be used in step b). The lubricant can be present from 0 to 30% by weight, preferably from 10 to 25% by weight, and more preferably from 10 to 15% by weight with respect to the total weight of softgel capsule composition.

Unexpectedly, the authors of the present invention have found that hydrophilic polyglycerols are suitable as lubricants for the production of the two molded halves, in particular of soft capsules, according to the rotary-die-method or another similar method of manufacturing molded halves made of a biopolymer.

The agent for the lubrication is water-soluble respectively water dispersible and comprises one or more polyglycerols, preferably consisting of 2 to 28 monomeric units. While many hydrophilic substances as for example glycerols, polyethylene glycols or propylene glycols are not suitable as lubricant, as they either lubricate insufficiently, seal insufficiently or cause undesirable changes of the gel matrix, the polyglycerols, as lubricants, combine very good sliding properties with a neutral behavior, in particular also towards a aqueous matrix as e.g. gelatin. Besides gelatin, it is possible to use also other biopolymers made to strips or extrusions, e.g. starch, starch derivatives, cellulose, cellulose derivatives, galactomannans, rubber kinds, agar-agar, and others as well as mixtures of those biopolymers. In particular, starch strips manufactured according to the method disclosed in US20020193452A1 can be used.

The triglyceride esters are desirably blended with from about 30 to about 87 weight percent vegetable oil, animal products, or mixtures thereof. These oil products must provide acceptable lubricity for the particular application, must be food-grade, must be foamable, and must be compatible so that the resultant lubricant is shelf-stable. Vegetable oils suitable for use are preferably selected from the group consisting of canola oil, corn oil, castor oil, soybean oil, jojoba oil, safflower oil, sunflower oil and palm oil, with canola oil and corn oil being particularly preferred. In place of all or a portion of the vegetable oil component of the invention, animal products such as tallow oil, lard oil, sperm oil, and mixtures thereof, can likewise be used in the foaming, food-grade lubricants of the invention. Similarly, some white mineral oil can be substituted for an equivalent percentage of vegetable oil in the blended lubricant if desired, although lubricity can be adversely affected by the use of too much white oil. The use of more triglyceride ester in the subject compositions may be needed for additional lubricity if white oil is substituted for a significant portion of the vegetable oil component. Foamability in the subject lubricants is preferably achieved by blending into the lubricant from about 2 to about 12 weight percent lecithin, with about 8 weight percent lecithin being particularly preferred. The amount and quality of the foam is generally proportional to the amount of lecithin used. The lecithin emulsifier used in the current invention should be food grade and is commercially available from many suppliers. There is no need for the lecithin to be chemically modified, although it should not contain an anti-foaming agent. One such product suitable for use in the invention is LECISOY^{®} N1. (US20060105094 A1).

In order to prepare the filler suspension for filling the softgel capsules according to the present invention, the above hormone formulation comprising micronized progesterone in combination with grapeseed oil has been prepared.

For the preparation of the hormone formulation, the first step is selecting micronized progesterone.

In the first variant, the hormone formulation further comprises soybean lecithin, and in the second variant the hormone formulation is free of soybean lecithin. The selection of the variant can be made in accordance with the allergenic requirement of the end pharmaceutical dosage form.

The filler suspension for obtaining the hormone formulation can be prepared by the following steps, which do not vary substantially by the presence or absence of soybean lecithin in the hormone formulation:
- blending grapeseed oil with soybean lecithin, when it is present, to get a homogeneous solution,
- adding micronized progesterone to the homogeneous solution, or adding micronized progesterone directly to the grapeseed oil in a formulation free of soybean lecithin,
- mixing micronized progesterone and grapeseed oil, and additionally with soybean lecithin when it is present, to get a homogenous suspension,
- deaerating filler suspension to remove entrapped air in order to obtain the filler suspension ready for use in a suitable equipment for encapsulating it into a softgel capsule shell.

In a third aspect, the present invention is directed to a pharmaceutical softgel capsule according to the first aspect of the present invention for use as a medicament.

The pharmaceutical softgel capsules according to the present subject matter can be administered orally or vaginally, according to the therapeutic indications.

The pharmaceutical softgel capsule according to the present subject matter is useful in the treatment of a physiological condition associated with progesterone deficiency.

Advantageously, the pharmaceutical softgel capsules of micronized progesterone in combination with grapeseed oil, with or without soybean lecithin, are useful in the treatment of a physiological condition associated with progesterone deficiency.

### Definitions

In the context of the present invention, the term "micronized progesterone" means a progesterone in which at least 99% of the particles have a particle size of less than 50 µm, preferably 50% of the particles have a particle size of between 1 and 10 µm, and even more preferentially 10% of the particles have a particle size of between 1 and 5 µm, these particle sizes being measured using a Malvern lase particle sizer according to a known method described in the methods of the present patent application.

In order to determine the particle size distributions of micronized, a sample of powder of progesterone was analyzed using laser and light scattering analysis. For micronized progesterone, D10, D50, D90 and D99 particle sizes were 1.36, 7.89, 18.5 µm and 29.8 µm, respectively.

### Brief Description of the Drawings

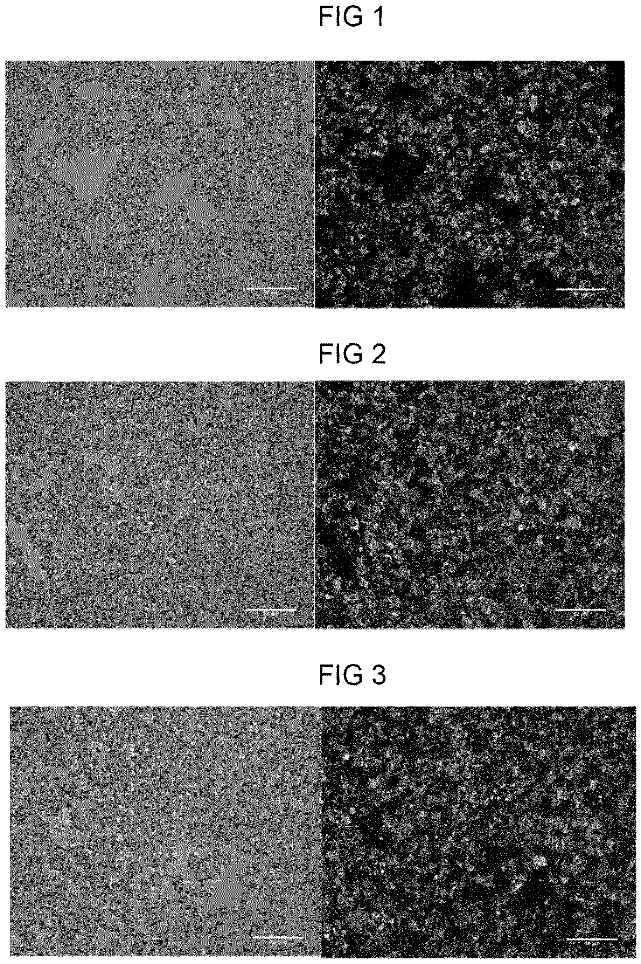

**Figure 1** shows a brightfield and polarized light microscopy observed under 40X of prior art (Utrogestan^{®}).
**Figure 2** shows a brightfield and polarized light microscopy observed under 40X of the softgel capsules according to Example 1 of the instant invention.
**Figure 3** shows a brightfield and polarized light microscopy observed under 40X of the softgel capsules according to Example 2 of the instant invention.
**Figure 4** depicts a graph of the dissolution test II of progesterone (%) *versus* the time (min) at a pH 4.5 of the softgel capsules according to Examples 1 and 2, compared with the softgel capsules of the prior art (Utrogestan^{®}).
**Figure 5** depicts a graph of the dissolution test III of progesterone (%) *versus* the time (min) at a pH 6.8 of the softgel capsules according to Examples 1 and 2, compared with the softgel capsules of the prior art (Utrogestan^{®}).

### Detailed Description of the Invention

Hereinafter, the best mode for carrying out the present invention is described in detail. Pharmaceutical softgel capsules according to the present invention comprise micronized progesterone in combination with grapeseed oil, with or without the presence of soybean lecithin.

The micronization of progesterone enhances absorption from the gastrointestinal tract. The micronization of progesterone decreases the average diameter of progesterone particles and thereby increases the surface area of the drug, facilitating aqueous dissolution in the small intestine. This could potentially result in greater drug bioavailability, the amount of drug reaching systemic circulation.

### Manufacturing process for preparing Progesterone softgel capsules 100 mg and 200 mg Preparation of the Gel Mass

In a best mode of gel mass preparation, a capsule shell composition comprising gelatin from 36 to 56% by weight, glycerine from 13.5 to 23.5% by weight, titanium oxide from 0.3 to 0.7% by weight, and water from 25 to 45% by weight, being the sum of said shell's components 100%, is used.

Physico-chemical properties of said capsule shell composition are included below.

| **Component** | **Defined Range (%)** | **Target (%)** | **Response** | **Obtained Range** | **Target** |
|---|---|---|---|---|---|
| Gelatin | 36 - 56 | 46 | Viscosity cPs | 3280 - 17250 | 7000-8500 |
| Glycerin | 13.5 - 23.5 | 18.5 | Hardness (before drying) (N) | 3.4 - 12.2 | >13-14 |
| TiO₂ | 0.3 - 0.7 | 0.5 | Hardness (after drying) (N) | 11.1 - 15.9 | |
| Water | 25 - 45 | 35 | Disintegration (min) | 5.39 - 9.11 | < 10 min |

The viscosity cPs of 7000-8500 in the table corresponds to 7-8.5 Pa.s .

For the preparation of the gel mass, purified water is added to a gelatin melter under vacuum. The temperature is maintained at 80°C (78° - 82°C) and gelatin is added to the melter. The gel mass is needed to be de-aerated to avoid bubbles presence.

A suspension of titanium dioxide in purified water is filtered through a 250 µm filter and added with continuous mixing to achieve uniform colour distribution. Glycerin is added and gel mass needs to be de-aerated to avoid bubbles presence.

The final preparation can be stored in the storage tanks at 50-55°C and prior to its usage for encapsulation the temperature can be increased at 60°C.

To complete the preparation of the gelatin suspension, the viscosity can be tested and adjusted to between 7 Pa.s (7000cP) and 8.5 Pa.s (8500cP).

The viscosity of the gelatin mass is a key parameter in the control of the shell formation and was controlled throughout the manufacture of each batch.

### Preparation of Progesterone Suspension (filler suspension)

For the preparation of the filler suspension, in the first variant, the hormone formulation comprises micronized progesterone 40% by weight, grapeseed oil 59.6% by weight, and soybean lecithin 0.4% by weight.

Soya lecithin and grapeseed oil are blended with continuous stirring until a homogenous solution is obtained. Micronized progesterone is added to the above mixture and blended until a homogeneous suspension is obtained. Fill suspension is de-aerated to remove the entrapped dissolved air.

For the preparation of the filler suspension, in the second variant, the hormone formulation comprises micronized progesterone 40% by weight and grapeseed oil 60% by weight. Micronized progesterone can be added to the grapeseed oil, and blended until a homogeneous suspension is obtained. Fill suspension is de-aerated to remove the entrapped dissolved air.

### Encapsulation

Softgel capsules are formed from two molded halves and filled with the filler suspension. This is achieved by feeding two gelatin ribbons between the two die-rolls, into cavity of which, the fill is injected.

The two ribbons can be made by feeding the capsule base mass (gelatin) in two spreader boxes installed on the left and on the right side of the machine. From there, the gelatin is dispersed over the two rotating casting drums where the gelatin ribbons are formed. The two gelatin ribbons, after travelling on the drums, are picked off by rollers and pass between lubricating oil rollers.

The surface of the ribbon that will be on the inside of the capsules is lubricated with medium chain triglyceride oil and the surface of the ribbon that will be on the outside of the capsules is lubricated with medium chain triglyceride oil including soybean lecithin, if apply.

Finally, the capsules are formed by the passage of the two ribbons through two identical rotating die rollers which contain holes around their circumference.

Preferably, the lubricant is medium-chain triglycerides (MCT), whose fatty acids have an aliphatic tail of 6-12 carbon atoms, which act as mold processing lubricants.

Other suitable mold processing lubricants or glidants for use herein, include but are not limited to, stearyl alcohol, stearic acid, glycerol monosterate (GMS), talc, magnesium stearate, silicon dioxide, amorphous silicic acid, and fumed silica; polyglycerols (preferably consisting of 2 to 28 monomeric units), starch derivatives and combinations or mixtures thereof. This functions primarily as a flow promoter for the composition. A preferred lubricant is stearyl alcohol, or GMS. A commercial grade of stearyl alcohol, such as Crodacol S95 (Croda Oleochemicals) is preferred for use herein.

Foamability in the subject lubricants is preferably achieved by blending into the lubricant from about 2 to about 12 weight percent lecithin, with about 8 weight percent lecithin being particularly preferred. The amount and quality of the foam is generally proportional to the amount of lecithin used. The lecithin emulsifier should be food or pharma grade and is commercially available from many suppliers. There is no need for the lecithin to be chemically modified, although it should not contain an anti-foaming agent.

### Drying

The filled capsules can be semi-dried using a tumbler conveyor process. The semi-dried capsules can be distributed in trays and dried in the drying tunnel maintained at controlled temperature and humidity to achieve desired moisture content and capsule hardness of greater than 13N in Progesterone 100 mg and 14N in Progesterone 200 mg was obtained.

### Inspection

The dried capsules were visually inspected by a camera to remove any possible defect.

### Examples

Hereinafter, the present invention is described in more detail and specifically with reference to the Examples and Figures, which however are not intended to limit the present invention. Table 1 shows the capsule shell components for preparing the gelatin mass of Examples 1 and 2.

**Table 1**

| **Component** | **Concentration (%)** | **Amount (Kg)** |
|---|---|---|
| Gelatin 150 bloom | 42.84 | 75.9 |
| Glycerol synthetic 99% | 17.34 | 30.72 |
| TiO₂ | 0.485 | 0.86 |
| Water | 39.34 | 69.7 |

Table 2 shows the progesterone formulation components for preparing the filler solution of Example 1.

**Table 2**

| **Component** | **Concentration (%)** | **Amount (Kg)** |
|---|---|---|
| Progesterone micronized | 40 | 20.00 |
| Grapeseed oil | 60 | 30.00 |

The lubricant used for making the molded halves of capsule-shaped shell in this example is MCT (10 to 15 kg per batch depending on encapsulation time).

Table 3 shows the progesterone formulation components for preparing the filler suspension of Example 2.

**Table 3**

| **Component** | **Concentration (%)** | **Amount (Kg)** |
|---|---|---|
| Progesterone micronized | 40 | 20.00 |
| Grapeseed oil | 59.6 | 29.80 |
| Soybean lecithin | 0.4 | 0.200 |

The lubricant used for making the molded halves of capsule-shaped shell in this example is MCT + soybean lecithin (10 to 15 kg per batch depending on encapsulation time containing at least (about) 0.5% of soybean lecithin, preferable 0.1%).

Table 4 below shows the particle size distribution of micronized progesterone, Example 1, Example 2 and prior art (Utrogestan ^{®}).

**Table 4**

| Particle size distribution | | | |
|---|---|---|---|
| | Dx(10) (µm) | Dx(50) (µm) | Dx(90) (µm) |
| Progesterone micronized to formulate Example 1 and 2 | 1.86 | 7.89 | 18.5 |
| Prior art (Utrogestan^{®}) | 4,24 | 9,99 | 18 |
| Example 1 | 2,28 | 8,47 | 15,98 |
| Example 2 | 5,53 | 13,86 | 27,2 |

The average diameter of particles sizes were determined by light diffraction (Mastersizer 3000, Malvern) using the refraction index of 1.33, which forms the dispersed phase in water with 0.1 % (p/v) of triton X-100. The samples were put separately into the sampling apparatus containing distilled water and rotating at 1800 rpm, and they were immediately analyzed with regard to the particle size. The optical conditions for the material used was refraction index of 1.520 and absorption index of 1.0.

### Example 1

### Preparation of the Progesterone suspension

The progesterone suspension preparation for a batch size of 100,000 capsules of 200 mg strength (50 Kg of filler).

Progesterone (20.0 kg) was incorporated into a stainless tank containing the grapeseed oil (30 kg) and mixed for at least 30 minutes to achieve a homogeneous suspension.

Degassing under vacuum was performed after the completion of the mixing process. The suspension was then filtered through a 600 µm sieve to de-agglomerate any poorly dispersed material.

### Preparation of the Gel Mass

Purified water (69.7 kg) was added to a gelatin melter under vacuum. The temperature was maintained at 80°C (78° - 82°C) and gelatin (75.9 kg) was added to the melter. The gel mass was de-aerated to avoid bubbles presence.

A suspension of titanium dioxide (0.86 kg) in purified water (5 kg) was filtered through a 250 µm filter and added with continuous mixing to achieve uniform color distribution. Glycerin (30.72kg) has to be added also and gel mass was de-aerated to avoid bubbles presence. The final preparation was stored in the storage tanks at 50-55°C and prior to its usage for encapsulation the temperature is increased at 60 °C.

To complete the preparation of the gelatin suspension, the viscosity was tested and adjusted to between 7 Pa.s (7000cP) and 8.5 Pa.s (8500cP).

### Encapsulation

Softgel capsules are formed from two molded halves and filled with the medicament concurrently. This is achieved by feeding two gelatin ribbons between the two die-rolls, into cavity of which, the fill is injected.

The two ribbons are made by feeding the capsule base mass (gelatin) in two spreader boxes installed on the left and on the right side of the machine. From there, the gelatin is dispersed over the two rotating casting drums where the gelatin ribbons are formed. The two gelatin ribbons, after travelling on the drums, are picked off by rollers and pass between lubricating oil rollers.

The surface of the ribbon that will be on the inside of the capsules is lubricated with medium chain triglyceride oil and the surface of the ribbon that will be on the outside of the capsules is lubricated with medium chain triglyceride oil including soya lecithin if apply.

The capsules are formed by the passage of the two ribbons through two identical rotating die rollers which contain holes around their circumference.

### Drying

The filled capsules were semi-dried using a tumbler conveyor process. The semi-dried capsules were distributed in trays and dried in the drying tunnel maintained at controlled temperature and humidity to achieve desired moisture content and capsule hardness of greater than 13N in Progesterone 100 mg and 14N in Progesterone 200 mg was obtained.

### Inspection

The dried capsules were visually inspected by a camera to remove any possible defect.

### Example 2

### Preparation of the Progesterone suspension

The progesterone suspension preparation for a batch size of 100,000 capsules of 200 mg strength (50 Kg of filler).

In a stainless tank, grapeseed oil (29.8 kg) and soy lecithin (0.2 kg) were mixed well until completely dissolved. Progesterone (20.0 kg) was incorporated into the same tank containing the grapeseed oil-soya lecithin mixture and mixed for at least 30 minutes to achieve a homogeneous suspension. Degassing under vacuum was performed after the completion of the mixing process. The suspension was then filtered through a 600 µm sieve to de-agglomerate any poorly dispersed material.

Preparation of the gel mass, encapsulation, drying and inspection were carried out as described in Example 1.

Dissolutions profiles of the softgel capsules prepared according to Examples 1 and 2 at two different test conditions were obtained and compared with the marketed softgel capsule of progesterone (Utrogestan^{®}).

Each capsule of Utrogestan^{®} (white capsules soft) contains 100 or 200 mg of micronized progesterone (INN). Excipients are: Arachis oil, soya lecithin, gelatin glycerol and titanium dioxide.

Two methods to test the dissolution were carried out and the conditions are as follows:

**Table TEST II**

| **Test conditions** (II) |
|---|
| 900 ml phosphate buffer pH 4,5 + 5% SDS |
| Apparatus USP TEST II Paddles |
| TIME: 30, 60, 90, 120, 150, 180 and 240 min |
| 150 rpm |

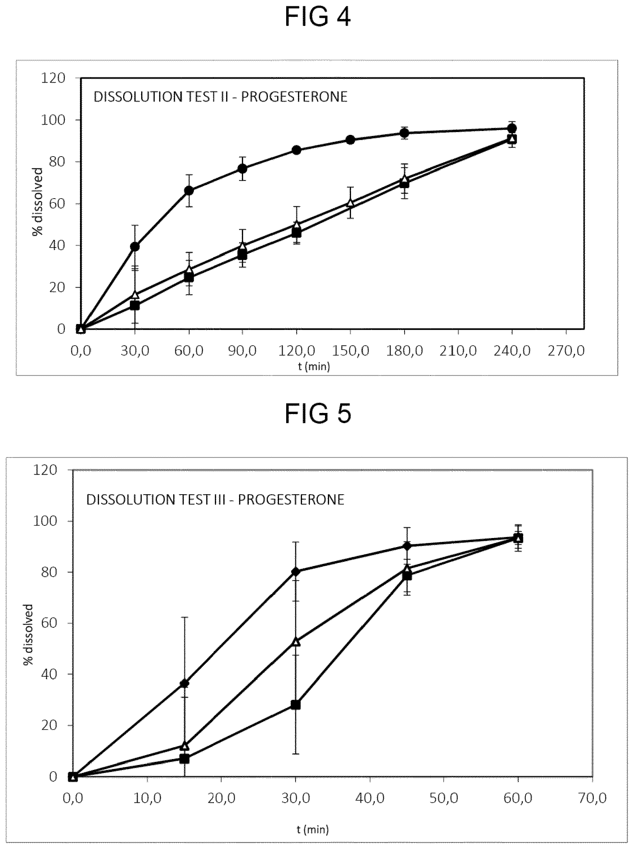

Fig. 4 depicts the dissolution of progesterone from the softgel capsules prepared according to Example 1, Example 2 and Utrogestan^{®}. The Y-axis indicates the amount dissolved, and the X-axis indicates the dissolution testing (according to the test conditions II, indicated in the Table above) time in minutes, wherein Δ indicates Example 1; ● indicates Example 2; and ■ indicates Utrogestan ^{®}.

**Table TEST III**

| **Test conditions (III)** |
|---|
| 250 ml phosphate buffer pH 6,8 + 4% SDS |
| Apparatus USP TEST III |
| TIME: 15, 30, 45 and 60 min |
| 30 dips/min. |
| JP SINKER |

Fig. 5 depicts the dissolution of progesterone from the softgel capsules prepared according to Example 1, Example 2 and Utrogestan ^{®}. The Y-axis indicates the amount dissolved, and the X-axis indicates the dissolution testing (according to the test conditions III, indicated in the Table above) time in minutes, wherein Δ indicates Example 1; ● indicates Example 2; and ■ indicates Utrogestan ^{®}.

Comparatively, from Figure 4, it can be seen that the softgel capsule of progesterone according to Example 1, which is free of soybean lecithin, shows at pH 4.5 a good dissolution rate, similar to that of the prior art (Utrogestan^{®}), see Example 1 (Δ) compared with the prior art (Utrogestan ^{®}) (■).

Comparatively, from Figure 5 the softgel capsule of progesterone according to Example 1, which is free of soybean lecithin, shows at pH 6.8 a dissolution rate higher than of that of the prior art (Utrogestan^{®}), see Example 1 (Δ) compared with the prior art (Utrogestan ^{®}) (■). Also comparatively, from Figure 4, it can be seen that the softgel capsule of progesterone according to Example 2, which includes soybean lecithin, shows at pH 4.5 a dissolution rate higher than of that of the prior art (Utrogestan^{®}), see Example 2 (●) compared with the prior art (Utrogestan ^{®}) (■), and also it can be seen that at pH 6.8, the dissolution rate is higher than of that of the prior art (Utrogestan^{®}), see Example 2 (●) compared with the prior art (Utrogestan ^{®}) (■).

Therefore, the softgel capsule of micronized progesterone free or not of soybean lecithin reveals a good dissolution profile at two different pHs (pH 4.5, and 6.8).

Stability assays of softgel capsules obtained according to Examples 1 and 2 were performed to evaluate two different conditions *versus* the time. The results obtained were collected and included in the following Tables.

### ► Example 1 (Grape seed oil without Soya Lecithin)

### ICH Condition 25°C - 60%RH

| **TESTS** | | **ACCEPTANCE LIMITS** | **T_{zero}** | **3 months** | **6 months** |
|---|---|---|---|---|---|
| | | DATE | Aug-16 | Nov-16 | Feb-17 |
| Appearance | | Ovoid soft gelatin capsules, off-white coloured. | Ovoid soft gelatin capsules. off-white coloured. | Ovoid soft gelatin capsules. off-white coloured. | Ovoid soft gelatin capsules. off-white coloured. |
| Loss on drying | | ≤ 15.0 % | 7.7 | 5.9 | ongoing |
| Progesterone Assay (%) | | 90.0 - 110.0 % | 99.7 | 99.1 | 99.3 |
| Disintegration | | < 20 min. | 13 min. | 10 min. | 9 min. |
| Uniformity of dosage units: content uniformity Progesterone Related Substances (%) | | AY ≤ 15.0 | 4.8 (n=10) | - | - |
| | Unknown individual impurities | ≤ 0.2% | RRT 0.25 = 0.03 | RRT 0.25 = 0.03 | RRT 0.25 = 0.03 |
| | RRT | | RRT 0.73 = 0.14 | RRT 0.73 = 0.10 | RRT 0.73 = 0.12 |
| | | | | | RRT 2.86= 0.09 |
| | Total impurities | ≤ 1.0% | 0.17 | 0.13 | 0.24 |
| | LOD = 0.01%; LOQ = 0.02% | | | | |

### ICH Condition 40°C - 75%RH

| **TESTS** | | **ACCEPTANCE LIMITS** | **T_{zero}** | **1 month** | **3 months** | **6 months** |
|---|---|---|---|---|---|---|
| | | DATE | Aug-16 | Sep-16 | Nov-16 | Feb-17 |
| Appearance | | Ovoid soft gelatin capsules. off-white coloured. | Ovoid soft gelatin capsules, off-white coloured. | Ovoid soft gelatin capsules. off-white coloured. | Ovoid soft gelatin capsules, off-white coloured. | Ovoid soft gelatin capsules. off-white coloured. |
| Loss on drying | | ≤ 15.0 % | 7.7 | 6.0 | 5.8 | ongoing |
| Progesterone Assay (%) | | 90.0 - 110.0 % | 99.7 | 98.3 | 98.5 | 100.0 |
| Disintegration | | < 20 min. | 13 min. | 14 min. | 11 min. | 11 min . |
| Uniformity of dosage units: content uniformity Progesterone Related Substances (%) | | AV ≤ 15.0 | 4.8 (n=10) | - | - | 4.0 (n=10) |
| | Unknown individual impurities | ≤ 0.2% | RRT 0.25 = 0.03 | RRT 0.25 = 0.03 | RRT 0.25 = 0.02 | RRT 0.25 = 0.03 |
| | | | | | | RRT 0.30 = 0.02 |
| | | | | | | RRT 0.49 = 0.03 |
| | RRT | | RRT 0.73 = 0.14 | RRT 0.73 = 0.12 | RRT 0.73 = 0.10 | RRT 0.73 = 0.12 |
| | | | | | | RRT 2.84= 0.10 |
| | Total impurities | ≤ 1.0% | 0.17 | 0.15 | 0.12 | 0.29 |
| | LOD = 0.01%; LOQ = 0.02% | | | | | |

### ► Example 2 (Grape seed oil with Soya Lecithin)

### ICH Condition 25°C - 60%RH

| **TESTS** | | **ACCEPTANCE LIMITS** | **T_{zero}** | **3 months** | **6 months** |
|---|---|---|---|---|---|
| | | DATE | Ago-16 | Nov-16 | Feb-17 |
| Appearance | | Ovoid soft gelatin capsules. off-white coloured. | Ovoid soft gelatin capsules. off-white coloured. | Ovoid soft gelatin capsules. off-white coloured. | Ovoid soft gelatin capsules. off-white coloured. |
| Loss on drying | | ≤ 15.0 % | 7.8 | 5.8 | ongoing |
| Progesterone Assay (%) | | 90.0 - 110.0 % | 101.2 | 100.7 | 102.3 |
| Disintegration | | < 20 min. | 12 min. | 12 min. | 12 min. |
| Uniformity of dosage units: content uniformity | | AV ≤ 15.0 | AV = 1.7 (n=10) | - | - |
| Progesterone Related Substances (%) | | | | | |
| | Unknown individual impurities | ≤ 0.2% | RRT 0.25 = 0.02 | RRT 0.25 = 0.03 | RRT 0.25 = 0.02 |
| | RRT | | RRT 0.73 = 0.13 | RRT 0.73 = 0.11 | RRT 0.73 = 0.12 |
| | | | | | RRT 2.84 = 0.10 |
| | Total impurities | ≤ 1.0% | 0.15 | 0.14 | 0.24 |
| | LOD = 0.01%; LOQ = 0.02% | | | | |

### ICH Condition 40°C - 75%RH

| **TESTS** | | **ACCEPTANCE LIMITS** | **T_{zero}** | **1 month** | **3 months** | **6 months** |
|---|---|---|---|---|---|---|
| | | DATE | Aug-16 | Sep-16 | Nov-16 | Feb-17 |
| Appearance | | Ovoid soft gelatin capsules. off-white coloured. | Ovoid soft gelatin capsules. off-white coloured. | Ovoid soft gelatin capsules. off-white coloured. | Ovoid soft gelatin capsules, off-white coloured. | Ovoid soft gelatin capsules. off-white coloured. |
| Loss on drying | | ≤ 15.0 % | 7.8 | 5.6 | 6.1 | ongoing |
| Progesterone Assay (%) | | 90.0 - 110.0 % | 101.2 | 100.2 | 102.0 | 102.4 |
| Disintegration | | < 20 min. | 12 min. | 15 min. | 13 min. | 11 min. |
| Uniformity of dosage units: content uniformity | | AV ≤ 15.0 | AV = 1.7 (n=10) | - | - | AV = 2.0 (n=10) |
| Progesterone Related Substances (%) | | | | | | |
| | Unknown individual impurities | ≤ 0.2% | RRT 0.25 = 0.02 | RRT 0.25 = 0.03 | RRT 0.25 = 0.02 | RRT 0.25 = 0.02 |
| | | | RRT 0.73 = 0.13 | RRT 0.73 = 0.13 | RRT 0.73 = 0.10 | RRT 0.73 = 0.12 |
| | RRT | | | | | RRT 2.84 = 0.09 |
| | Total impurities | ≤ 1.0% | 0.15 | 0.16 | 0.12 | 0.23 |
| | LOD = 0.01%; LOQ = 0.02% | | | | | |

As described above in detail, the novel softgel capsule of progesterone according to the present invention has at least one of the following advantages:
- The softgel capsule of progesterone does not use peanut oil, which is particularly advantageous for using in mammals having a hypersensitive to the allergens.
- The softgel capsule of progesterone is free of soybean lecithin, which is more advantageous for using in mammals having higher hypersensitive to the allergens.

## Claims

1. A pharmaceutical hormone softgel capsule comprising an outer soft capsule shell and an inner hormone formulation, **characterized in that**:
- the outer soft capsule shell comprises gelatin, and
- the inner hormone formulation comprises micronized progesterone in combination with grapeseed oil.

2. Softgel capsule according to claim 1, wherein the hormone formulation further comprises soybean lecithin.

3. Softgel capsule according to any one of claims 1 or 2, wherein the hormone formulation comprises micronized progesterone 20-60% by weight, grapeseed oil 39-80% by weight, and soybean lecithin 1% by weight, preferably progesterone 40% by weight, grapeseed oil 59.6% by weight, and soybean lecithin 0.4% by weight.

4. Softgel capsule according to claim 1, wherein the hormone formulation is free of soybean lecithin.

5. Softgel capsule according to claim 4, wherein the hormone formulation comprises micronized progesterone 20-60% by weight in combination with grapeseed oil 40-80% by weight, preferably progesterone 40% by weight in combination with grapeseed oil 60% by weight.

6. Softgel capsule according to claim 1, wherein the outer soft capsule shell composition comprises gelatin between 36 to 56% by weight, glycerine between 13.5 to 23.5% by weight, titanium oxide between 0.3 to 0.7% by weight, and water between 25 to 45% by weight, being the sum of said shell's components 100%.

7. Softgel capsule according to claim 6, wherein the outer soft capsule shell composition comprises gelatin between 40 to 45% by weight, glycerine between 15 to 20% by weight, titanium oxide between 0.4 to 0.6% by weight, and water between 32 to 42% by weight, being the sum of said shell's components 100%.

8. A process for the preparation of a softgel capsule of micronized progesterone according to any one of claims 1 to 7, the process comprising selecting micronized progesterone, and separately, preparing a gelatin mass for making an outer soft capsule shell and a filler suspension as hormone formulation, **characterized in that** the process comprises the following steps:
- encapsulating the previously prepared filler suspension into the previously prepared softgel capsule shell,
- drying the encapsulated filler suspension at controlled conditions of temperature and humidity, and
- confirming moisture content and hardness of the thus prepared softgel capsules.

9. Process according to claim 8, wherein a gelatin mass composition which comprises gelatin, glycerine, titanium oxide, and water is processed in accordance with the following steps:
a) preparing a gelatin mass:
- heating gelatin in water to form a gel mass,
- deaerating the gelatin mass,
- adding into the gelatin mass an opacifying or colouring agent and glycerine to form a solution,
- deaerating the solution, and
- confirming viscosity of the solution, and adjusted if necessary to a value from 7 Pa.s (7000cP) and 8.5 Pa.s (8500cP),
b) preparing molded halves of capsule-shaped shell from the solution obtained in step a) by using a suitable equipment.

10. Process according to claim 9, wherein in step b) is employed a lubricant which may be present from 0.01 to 30% by weight, preferably from 10 to 25% by weight, and more preferably from 10 to 15% by weight with respect of the total weight of the softgel capsule composition.

11. Process according to claim 10, wherein the lubricant is a triglyceride ester.

12. Process according to claim 8, wherein the filler solution which comprises micronized progesterone and grapeseed oil, and optionally soybean lecithin, is processed in accordance with the following steps:
- blending grapeseed oil with soybean lecithin, when it is present, to get a homogeneous solution,
- adding micronized progesterone to the homogeneous solution, or adding micronized progesterone directly to the grapeseed oil in a formulation free of soybean lecithin,
- mixing micronized progesterone and grapeseed oil, and additionally with soybean lecithin when it is present, to get a homogenous suspension,
- deaerating filler suspension to remove entrapped air in order to obtain the filler suspension ready for use in a suitable equipment for encapsulating it into a softgel capsule shell.

13. A pharmaceutical softgel capsule according to any one of claims 1 to 7 for use as a medicament.

14. Pharmaceutical softgel capsule for use according to claim 13 for use in the treatment of a physiological condition associated with progesterone deficiency, in oral or vaginal dosage form.

## Patentansprüche

1. Pharmazeutische Hormon-Weichgelkapsel, umfassend eine äußere Weichkapselschale und eine innere Hormonzubereitung, **dadurch gekennzeichnet**
**dass** die äußere Weichkapselschale Gelatine umfasst, und
die innere Hormonzubereitung mikronisiertes Progesteron in Kombination mit Traubenkernöl umfasst.

2. Weichgelkapsel nach Anspruch 1, wobei die Hormonzubereitung ferner Sojabohnen-Lecithin umfasst.

3. Weichgelkapsel nach einem der Ansprüche 1 oder 2, wobei die Hormonzubereitung 20-60 Gew.-% mikronisiertes Progesteron, 39-80 Gew.-% Traubenkernöl und 1 Gew.-% Sojabohnen-Lecithin umfasst, vorzugsweise 40 Gew.-% Progesteron, 59,6 Gew.-% Traubenkernöl und 0,4 Gew.-% Sojabohnen-Lecithin.

4. Weichgelkapsel nach Anspruch 1, wobei die Hormonzubereitung frei von Sojabohnen-Lecithin ist.

5. Weichgelkapsel nach Anspruch 4, wobei die Hormonzubereitung 20-60 Gew.-% mikronisiertes Progesteron in Kombination mit 40-80 Gew.-% Traubenkernöl umfasst, vorzugsweise 40 Gew.-% Progesteron in Kombination mit 60 Gew.-% Traubenkernöl.

6. Weichgelkapsel nach Anspruch 1, wobei die äußere Weichkapselschalen-Zusammensetzung zwischen 36 bis 56 Gew.-% Gelatine, zwischen 13,5 bis 23,5 Gew.-% Glycerin, zwischen 0,3 bis 0,7 Gew.-%Titanoxid und zwischen 25 bis 45 Gew.-% Wasser umfasst, wobei die Summe der Schalenkomponenten 100 Gew.-% beträgt.

7. Weichgelkapsel nach Anspruch 6, wobei die äußere Weichkapselschalen-Zusammensetzung zwischen 40 bis 45 Gew.-% Gelatine, zwischen 15 bis 20 Gew.-% Glycerin, zwischen 0,4 bis 0,6 Gew.-%Titanoxid und zwischen 32 bis 42 Gew.-% Wasser umfasst, wobei die Summe der Schalenkomponenten 100 Gew.-% beträgt.

8. Verfahren zur Herstellung einer Weichgelkapsel mit mikronisierten Progesteron nach einem der Ansprüche 1 bis 7, wobei das Verfahren das Auswählen von mikronisiertem Progesteron und die getrennte Herstellung einer Gelatinemasse zur Bildung einer äußeren Weichkapselschale und einer Füllstoffsuspension als Hormonzubereitung umfasst, **dadurch gekennzeichnet, dass** das Verfahren die folgenden Stufen umfasst:
Einkapseln der vorher hergestellten Füllstoffsuspension in der vorher hergestellten Weichgelkapselschale,
Trocknen der eingekapselten Füllstoffsuspension bei kontrollierten Temperatur- und Feuchtigkeitsbedingungen und
Bestätigen von Feuchtigkeitsgehalt und Härte der so hergestellten Weichgelkapseln.

9. Verfahren nach Anspruch 8, wobei eine Gelatinemasse-Zusammensetzung, die Gelatine, Glycerin, Titanoxid und Wasser umfasst, gemäß den folgenden Stufen verarbeitet wird:
a) Herstellen einer Gelatinemasse:
- Erwärmen von Gelatine in Wasser zur Bildung einer Gelatinemasse,
- Entlüften der Gelatinemasse,
- Zugeben eines Trübungsmittels oder Färbungsmittels und Glycerin in die Gelatinemasse zur Bildung einer Lösung,
- Entlüften der Lösung und
- Bestätigen der Viskosität der Lösung und gegebenenfalls Einstellen auf einen Wert von 7 Pa.s (7000 cP) bis 8,5 Pa.s (8500 cP),
b) Herstellen von Formhälften einer kapselförmigen Schale aus der in Stufe a) erhaltenen Lösung unter Verwendung einer geeigneten Vorrichtung.

10. Verfahren nach Anspruch 9, wobei in Stufe b) ein Gleitmittel verwendet wird, das in einem Anteil von 0,01 bis 30 Gew.-%, vorzugsweise von 10 bis 25 Gew.-% und insbesondere von 10 bis 15 Gew.-%, bezogen auf das Gesamtgewicht der Weichgelkapsel-Zusammensetzung, vorliegt.

11. Verfahren nach Anspruch 10, wobei es sich beim Gleitmittel um einen Triglyceridester handelt.

12. Verfahren nach Anspruch 8, wobei die Füllstofflösung, die mikronisiertes Progesteron und Traubenkernöl und gegebenenfalls Sojabohnen-Lecithin umfasst, gemäß den folgenden Stufen verarbeitet wird:
- Vermischen von Traubenkernöl mit Sojabohnen-Lecithin, sofern dieses vorliegt, zur Bildung einer homogenen Lösung,
- Zugeben von mikronisiertem Progesteron zu der homogenen Lösung oder direktes Zugeben von mikronisiertem Progesteron zum Traubenkernöl in einer Zubereitung, die kein Sojabohnen-Lecithin umfasst,
- Vermischen von mikronisiertem Progesteron und Traubenkernöl und zusätzlich von Sojabohnen-Lecithin, sofern dieses vorliegt, zur Bildung einer homogenen Suspension,
- Entlüften von Füllstoffsuspension zur Entfernung von eingeschlossener Luft, um die Füllstoffsuspension zu erhalten, die zur Verwendung beim Verkapseln derselben in einer geeigneten Vorrichtung in eine Weichgelkapsel gebrauchsfertig ist.

13. Pharmazeutische Weichgelkapsel nach einem der Ansprüche 1 bis 7 zur Verwendung als Arzneimittel.

14. Pharmazeutische Weichgelkapsel zur Verwendung nach Anspruch 13 zur Behandlung eines physiologischen Zustands im Zusammenhang mit Progesteronmangel in oraler oder vaginaler Darreichungsform.

## Revendications

1. Capsule molle d'hormone pharmaceutique comprenant une enveloppe externe de capsule molle et une formulation hormonale interne, **caractérisée en ce que**:
- l'enveloppe externe de la capsule molle comprend de la gélatine, et
- la formulation hormonale interne comprend de la progestérone micronisée en combinaison avec de l'huile de pépins de raisin.

2. Capsule molle selon la revendication 1, dans laquelle la formulation hormonale comprend en outre de la lécithine de soja.

3. Capsule molle selon l'une quelconque des revendications 1 ou 2, dans laquelle la formulation hormonale comprend de la progestérone micronisée entre 20 et 60 % en poids, de l'huile de pépins de raisin entre 39 et 80% en poids, et de la lécithine de soja à 1 % en poids, de préférence de la progestérone à 40% en poids, de l'huile de pépins de raisin à 59,6% en poids, et de la lécithine de soja à 0,4% en poids.

4. Capsule molle selon la revendication 1, dans laquelle la formulation hormonale est exempte de lécithine de soja.

5. Capsule molle selon la revendication 4, dans laquelle la formulation hormonale comprend de la progestérone micronisée entre 20 et 60% en poids en combinaison avec de l'huile de pépins de raisin entre 40 et 80% en poids, de préférence de la progestérone à 40% en poids en combinaison avec de l'huile de pépins de raisin à 60% en poids.

6. Capsule molle selon la revendication 1, dans laquelle la composition de l'enveloppe externe de la capsule molle comprend de la gélatine entre 36 et 56% en poids, de la glycérine entre 13,5 et 23,5% en poids, de l'oxyde de titane entre 0,3 et 0,7% en poids, et de l'eau entre 25 et 45% en poids, la somme des composants de ladite enveloppe étant de 100%.

7. Capsule molle selon la revendication 6, dans laquelle la composition de l'enveloppe externe de la capsule molle comprend de la gélatine entre 40 et 45% en poids, de la glycérine entre 15 et 20% en poids, de l'oxyde de titane entre 0,4 et 0,6% en poids, et de l'eau entre 32 et 42% en poids, la somme des composants de ladite enveloppe étant de 100%.

8. Procédé de préparation d'une capsule molle de progestérone micronisée selon l'une quelconque des revendications 1 à 7, le procédé comprenant la sélection de la progestérone micronisée, et séparément, la préparation d'une masse de gélatine pour fabriquer une enveloppe externe de capsule molle et une suspension de charge en tant que formulation hormonale, **caractérisé en ce que** le procédé comprend les étapes suivantes:
- encapsuler la suspension de charge préalablement préparée dans l'enveloppe de la capsule molle préalablement préparée,
- sécher la suspension de charge encapsulée dans des conditions contrôlées de température et d'humidité, et
- confirmer la teneur en eau et la dureté des capsules molles ainsi préparées.

9. Procédé selon la revendication 8, dans lequel une composition de masse de gélatine qui comprend de la gélatine, de la glycérine, de l'oxyde de titane et de l'eau est traitée selon les étapes suivantes:
a) préparer une masse de gélatin:
- chauffer la gélatine dans de l'eau pour former une masse de gel,
- désaérer la masse de gélatine,
- ajouter à la masse de gélatine un agent opacifiant ou colorant et de la glycérine pour former une solution,
- désaérer la solution, et
- confirmer la viscosité de la solution, et l'ajuster si nécessaire à une valeur comprise entre 7Pa.s (7000cP) et 8,5Pa.s (8500cP),
b) préparer des moitiés moulées d'enveloppe en forme de capsule à partir de la solution obtenue à l'étape a) en utilisant un équipement approprié.

10. Procédé selon la revendication 9, dans lequel, à l'étape b), on utilise un lubrifiant qui peut être présent entre 0,01 et 30% en poids, de préférence entre 10 et 25% en poids, et plus préférablement entre 10 et 15% en poids par rapport au poids total de la composition de capsule molle.

11. Procédé selon la revendication 10, dans lequel le lubrifiant est un ester de triglycéride.

12. Procédé selon la revendication 8, dans lequel la solution de charge qui comprend de la progestérone micronisée et de l'huile de pépins de raisin, et éventuellement de la lécithine de soja, est traitée selon les étapes suivantes:
- mélanger l'huile de pépins de raisin avec la lécithine de soja, lorsqu'elle est présente, pour obtenir une solution homogène,
- ajouter de la progestérone micronisée à la solution homogène, ou ajouter de la progestérone micronisée directement à l'huile de pépins de raisin dans une formulation exempte de lécithine de soja,
- mélanger la progestérone micronisée et l'huile de pépins de raisin, ainsi que la lécithine de soja lorsqu'elle est présente, pour obtenir une suspension homogène,
- désaérer la suspension de charge pour éliminer l'air piégé afin d'obtenir la suspension de charge prête à être utilisée dans un équipement approprié pour l'encapsuler dans une enveloppe de capsule molle.

13. Capsule molle pharmaceutique selon l'une quelconque des revendications 1 à 7 destinée à être utilisée comme médicament.

14. Capsule molle pharmaceutique à utiliser selon la revendication 13 pour une utilisation dans le traitement d'un état physiologique associé à une déficience en progestérone, sous forme posologique orale ou vaginale.
